# EUROPEAN PATENT APPLICATION

(11) **EP 2 407 455 A1**
(43) Date of publication of application: **18.01.2012**
(21) Application number: 10753521.3
(22) Date of filing: 16.03.2010
(51) Int. Cl.: C07D 209/44, A61K 51/00

(54) **MOLECULAR PROBE PRECURSOR FOR PANCREATIC ISLET IMAGING AND USE OF SAME**

(30) Priority: 19.03.2009 JP 2009068837; 07.08.2009 US 272024
(71) Applicant: Kyoto University, Sakyo-ku Kyoto-shi Kyoto 606-8501 (JP); Arkray, Inc., Minami-ku Kyoto-shi Kyoto 601-8045 (JP)
(72) Inventor: INAGAKI, Nobuya, Kyoto 606-8501 (JP); SAJI, Hideo, Kyoto-shi Kyoto 606-8501 (JP); TOYODA, Kentaro, Kyoto-shi Kyoto 606-8501 (JP); KIMURA, Hiroyuki, Kyoto-shi Kyoto 606-8501 (JP); HIRAO, Konomu, Kyoto-shi Kyoto 601-8045 (JP); NAGAKAWA, Kenji, Kyoto-shi Kyoto 601-8045 (JP)
(74) Representative: Golding, Louise Ann
(86) International application number: PCT/JP2010/054449
(87) International publication number: WO 2010/107029

(57) **Abstract**

A precursor of a molecular probe for imaging of pancreatic islets is a compound expressed as the following formula (I): 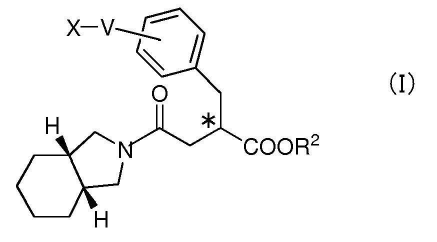 wherein -V-X represents a substituent on a benzene ring, V represents a bond, R¹, or OR¹, R¹ represents a C₁-C₆ alkylene group, R² represents H (hydrogen atom), a C₁-C₆ alkyl group, a C₇-C₁₀ aralkyl group, or a protecting group, X represents a OMs group, a OTs group, a OTf group, Br (bromine atom), or I (iodine atom), and carbon marked with ^{*} is an asymmetric carbon atom.

## Description

### Technical Field

The present invention relates to a precursor of a molecular probe for imaging of pancreatic islets and the use of the same.

### Background Art

At present, approximately more than 8.2 million people suffer from type 2 diabetes in Japan, and the number of diabetic patients is increasing steadily. To deal with this problem, an intervention based on a glucose tolerance test before the onset of diabetes has been carried out, but not been fully effective yet. This is because a pancreatic islets disorder has already progressed significantly by the time of the borderline stage in which the glucose tolerance test reveals a dysfunction, and thus the starting time of the intervention may be late.

The amount of pancreatic islets (particularly the amount of pancreatic β cells) is reduced prior to abnormal glucose tolerance during the development of diabetes. Therefore, the treatment of diabetes is already difficult when the dysfunction is detected and the patient is conscious of it. Various medications such as a rapid-acting insulin secretagogue and a sulfonylurea (SU) agent are commercially available as antidiabetic agents. For example, there are two types of rapid-acting insulin secretagogues on the market: nateglinide and mitiglinide. Mitiglinide ((+)-monocalcium bis [(2S, 3a,
7a-cis)-α-benzylhexahydro-γ-oxo-2-isoindolinebutyrate] dihydrate (molecular formula: C₃₈H₄₈CaN₂O₂·2H₂O, molecular weight: 704.91)) is a ligand of SUR1 (sulfonylurea receptor) constituting a part of a K channel of the pancreatic β cells. The ingestion of mitiglinide closes the potassium channel regardless of ATP and has the effect of promoting insulin secretion. Therefore, mitiglinide has been used widely as an antidiabetic agent (Patent documents 1 and 2).

On the other hand, if a reduction in the amount of pancreatic islets and/or pancreatic β cells can be found early there is a possibility that diabetes is able to be prevented and treated. Thus, a noninvasive technique for imaging of pancreatic islets, in particular a noninvasive technique for imaging of pancreatic islets to measure the amount of pancreatic islets and/or pancreatic β cells is desired for the prevention and diagnosis of diabetes. Among others, a molecular probe capable of imaging the pancreatic islets, and preferably capable of imaging the pancreatic β cells is desired especially

### Prior Art Documents

### Patent Documents

Patent document 1: Japanese Patent 2686863 B
Patent document 2: Japanese Patent 2686879 B

### Disclosure of Invention

### Problem to be Solved by the Invention

With the foregoing in mind, it is an object of the present invention to provide a molecular probe for imaging of pancreatic islets that enables three-dimensional noninvasive imaging of pancreatic islets.

### Means for Solving Problem

The present invention is directed to a precursor of a molecular probe for imaging of pancreatic islets. The precursor is expressed as the following formula (I): 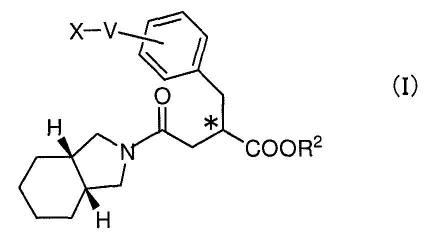 wherein -V-X represents a substituent on a benzene ring, V represents a bond, R¹, or OR⁰, R¹ represents a C₁-C₆ alkylene group, R² represents H (hydrogen atom), a C₁-C₆ alkyl group, a C₇-C₁₀ aralkyl group, or a protecting group, X represents a mesylate (OMs) group, a tosylate (OTs) group, a triflate (OTf) group, Br (bromine atom), or I (iodine atom), and carbon marked with ^{*} is an asymmetric carbon atom. The molecular probe is used for imaging of pancreatic islets.

### Effects of the Invention

The precursor of a molecular probe for imaging of pancreatic islets of the present invention enables imaging of pancreatic islets, preferably three-dimensional imaging of pancreatic islets, and more preferably noninvasive imaging of pancreatic islets, e.g., by positron emission tomography (PET).

### Brief Description of Drawings

[FIG. 1] FIGS. 1A and 1B are graphs showing an example of the results of changes in biodistribution of a molecular probe of the present invention over time.
[FIG. 2] FIG. 2 is a graph showing an example of the result of a receptor binding experiment in Example.
[FIG. 3] FIG. 3 is a diagram showing an example of the result of imaging of pancreatic islets (PET) in Example.

### Description of the Invention

It is known that the selectivity of mitiglinide for SUR1 in vitro is about 100 times higher than that of nateglinide, which is the same type of rapid-acting insulin secretagogue. Moreover, mitiglinide is known to have high selectivity for the pancreatic β cells. However, it is unclear whether mitiglinide labeled with a positron-emitting nuclide behaves in the same way.

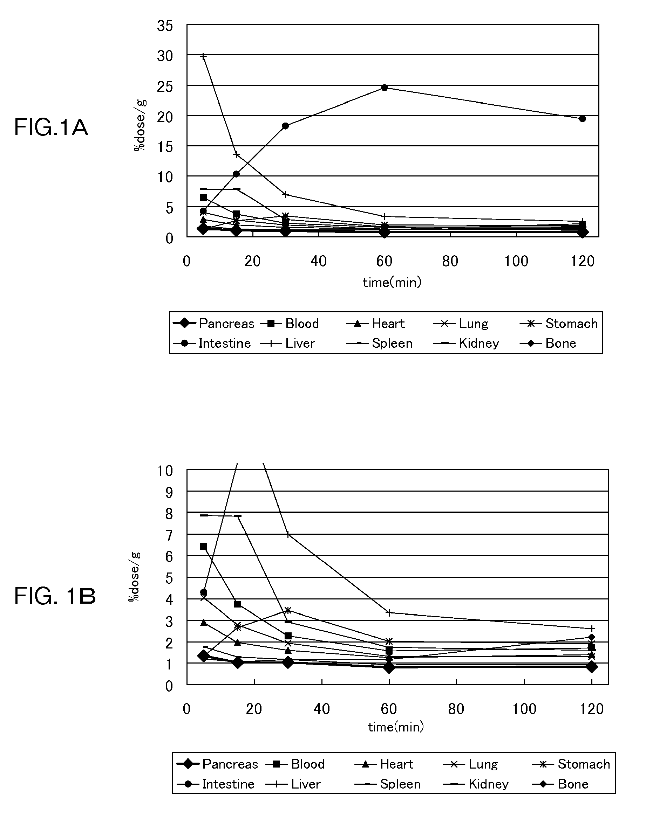

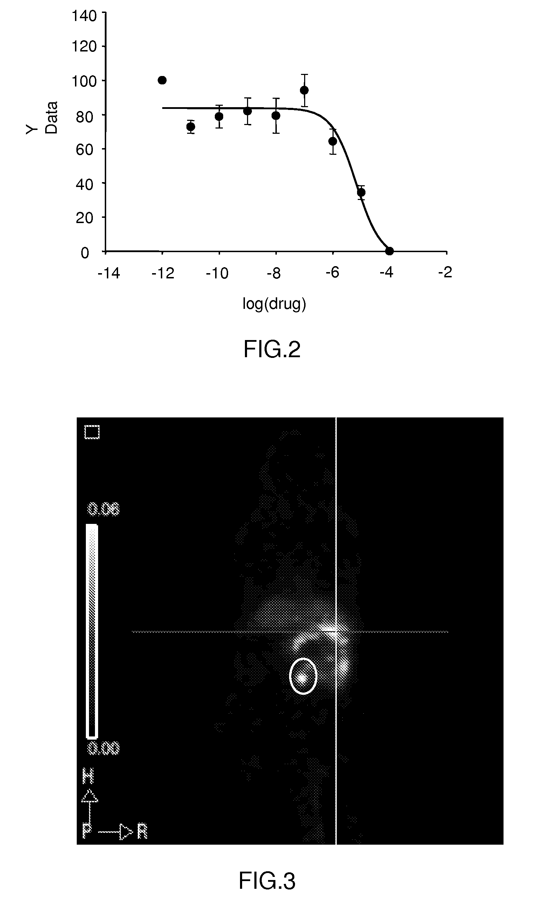

Actually, when X of the compound expressed as the formula (I) was labeled with the positron-emitting nuclide, and the biodistribution in a mouse was investigated, a satisfactory result was not obtained, as shown in FIGS. 1A and 1B of the present specification. Also, the receptor binding experiment did not show a particularly excellent binding capacity, as shown in FIG. 2 of the present specification. In this case, although the availability of the molecular probe was not suggested, the present inventors captured images using the compound expressed as the formula (I) in which X was labeled with the positron-emitting nuclide, and surprisingly found that those images were extremely good. The present invention is based on the findings. That is, the present invention preferably can perform three-dimensional noninvasive imaging of pancreatic islets. Moreover, the present invention preferably can perform quantitative imaging of pancreatic islets, and more preferably can achieve both quantitative imaging and three-dimensional noninvasive imaging of pancreatic islets, which have been difficult so far.

The present invention can perform the three-dimensional imaging of pancreatic islets, and therefore preferably can measure the amount of pancreatic islets. Moreover, the present invention can perform the noninvasive imaging of pancreatic islets, and therefore preferably can be applied to the test and diagnosis in humans. Accordingly, the present invention preferably can provide the prevention, treatment, and diagnostic methods of diabetes based on the measurement of the amount of pancreatic islets.

As described above, it is known that the amount of pancreatic islets is reduced prior to abnormal glucose tolerance during the development of diabetes. Therefore, due to the imaging of pancreatic islets and/or the measurement of the amount of pancreatic islets, a minute change in pancreatic islets can be found, e.g., before the onset or in the early stage of diabetes. This makes it possible to detect and diagnose diabetes in the very early stage. Thus, the precursor of a molecular probe for imaging of pancreatic islets of the present invention is useful for early detection and diagnosis of diabetes, and preferably for very early detection and diagnosis of diabetes.

The present invention is directed to the following:
[1] a precursor of a molecular probe for imaging of pancreatic islets (also referred to as a "molecular probe precursor of the present invention" in the following) that is expressed as the following formula (I): 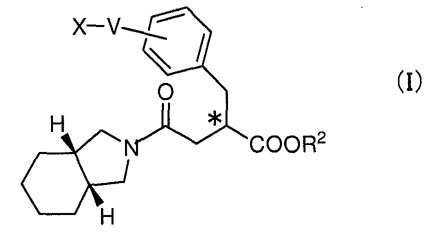 wherein -V-X represents a substituent on a benzene ring, V represents a bond, R¹, or OR⁰, R¹ represents a C₁-C₆ alkylene group, R² represents H (hydrogen atom), a C₁-C₆ alkyl group, a C₇-C₁₀ aralkyl group, or a protecting group, X represents a mesylate (OMs) group, a tosylate (OTs) group, a triflate (OTf) group, Br (bromine atom), or I (iodine atom), and carbon marked with ^{*} is an asymmetric carbon atom,
   wherein the molecular probe is used for imaging of pancreatic islets;
[2] the precursor according to [1], wherein in the formula (I), R¹ represents an ethylene group, a propylene group, or a butylene group, R² represents H, a methyl group, or a protecting group, and -V-X is in a meta position or a para position;
[3] the precursor according to [1] or [2], wherein in the formula (I), the protecting group is a protecting group of a carboxyl group;
[4] the precursor according to any one of [1] to [3], wherein the precursor is used for noninvasive imaging of pancreatic islets;
[5] the precursor according to any one of [1] to [4], wherein the precursor is used for imaging of pancreatic islets to quantify an amount of pancreatic islets;
[6] the precursor according to any one of [1] to [5], wherein the precursor is used for imaging of pancreatic islets to prevent, treat, or diagnose diabetes;
[7] the precursor according to any one of [1] to [6], wherein the imaging of pancreatic islets is performed by positron emission tomography (PET);
[8] a molecular probe for imaging of pancreatic islets that is used for imaging of pancreatic islets and expressed as the following formula (II): 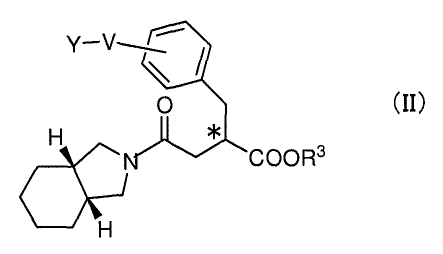 wherein -V-Y represents a substituent on a benzene ring, V represents a bond, R¹, or OR¹, R¹ represents a C₁-C₆ alkylene group, R³ represents H (hydrogen atom), a C₁-C₆ alkyl group, or a C₇-C₁₀ aralkyl group, Y represents ¹¹C,¹⁵O,¹⁸F, ^{99m}Tc, ¹¹¹In, or ¹²³I, and carbon marked with ^{*} is an asymmetric carbon atom;
[9] the molecular probe according to [8], wherein the molecular probe is obtained by labeling the precursor according to any one of [1] to [7];
[10] a method for imaging pancreatic islets including labeling the precursor according to any one of [1] to [7];
[11] a method for measuring an amount of pancreatic islets including preparing a molecular probe for imaging of pancreatic islets by labeling the precursor according to any one of [1] to [7], and calculating the amount of pancreatic islets from a result of the imaging of pancreatic islets using the molecular probe;
[12] a method for producing a molecular probe for imaging of pancreatic islets including labeling the precursor according to any one of [1] to [7]; and
[13] a kit for preparing a molecular probe for imaging of pancreatic islets including the precursor according to any one of [1] to [7].

### [Imaging of pancreatic islets]

In the present invention, the imaging of pancreatic islets is defined as molecular imaging of pancreatic islets and includes imaging of a spatial and/or temporal distribution of pancreatic islets in vivo. Moreover, in the present invention, preferred target molecules for the imaging of pancreatic islets are pancreatic β cells in view of the prevention, treatment, and diagnosis of diabetes. Further, in the present invention, it is preferable that the imaging of pancreatic islets is three-dimensional noninvasive imaging in view of the quantification of the amount of pancreatic islets and the application to humans. The method for imaging is not particularly limited as long as it allows the pancreatic islets to be imaged noninvasively Examples of the imaging method include positron emission tomography (PET), single photon emission computed tomography (SPECT), magnetic resonance imaging (MRI), and a method using X-rays, visible light, fluorescence, near infrared light, ultrasonic waves, or the like. Among them, the PET is preferred in view of using the molecular probe precursor of the present invention to quantify the amount of pancreatic islets.

### [Molecular probe precursor of the present invention]

The molecular probe precursor of the present invention is a precursor of a molecular probe used for imaging of pancreatic islets and includes a compound expressed as the following formula (I). The molecular probe precursor of the present invention is a compound that can be bound to the pancreatic islets, preferably to the pancreatic β cells, and more preferably to SUR1 on the pancreatic β cells after labeling.

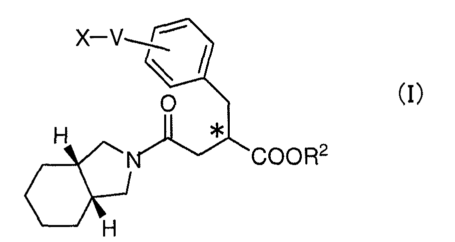

In the formula (I), V represents a bond, R¹, or OR¹, and R¹ represents a C₁-C₆ alkylene group. In the present invention, the "bond" means that X is bound directly to the benzene ring without the intervention of other atoms. In the present invention, the "C₁-C₆ alkylene group" means an alkylene group having 1 to 6 carbon atoms and can be a linear or branched alkylene group such as a methylene group, an ethylene group, a propylene group, a butylene group, a pentylene group, a hexylene group, or the like. In particular, the ethylene group, the propylene group, or the butylene group is preferred, and the ethylene group or the propylene group is more preferred. Examples of V include the bond, -C₂H₂-, -C₃H₆-, -O-C₂H₂-, and -O-C₃H₆-. The molecular probe precursor of the present invention can be bound to the pancreatic islets, preferably can be bound to the pancreatic β cells in view of the quantification of the amount of pancreatic islets and the test and diagnostic applications, more preferably is specific to at least the pancreatic β cells in the pancreas, and further preferably is specific to the extent that the signals from the pancreatic β cells are not superimposed on those from the other organs and tissues at least inside the human body.

In the formula (I), R² represents H, a C₁-C₆ alkyl group, a C₇-C₁₀ aralkyl group, or a protecting group. The C₁-C₆ alkyl group is as described above for R¹. In the present invention, the "C₇-C₁₀ axalkyl group" means an aralkyl group having 7 to 10 carbon atoms and can be a benzyl group, a phenethyl group, or the like. R² is preferably H, a methyl group, the benzyl group, or the protecting group, and more preferably H, the methyl group, or the protecting group. The protecting group can be a known protecting group of a carboxyl group. For example, the protecting group can be tert-butyl ester, benzil (Bn), benzoyl (Bz), methyl ethyl ester, a silyl protecting group, or the like. Examples of the silyl protecting group include trimethylsilyl (TMS), triethylsilyl (TES), tert-butyldiphenylsilyl (TBDPS), and tert-butyldimethylsilyl (TBDMS).

In the formula (I), -V-X represents a substituent on the benzene ring, preferably is in the ortho position, the meta position, or the para position, and more preferably is in the meta position or the para position in view of the affinity for a receptor. In the formula (I), X represents the OMs group, the OTs group, the OTf group, Br, or I, and preferably the OTs group, Br, or I. In the formula (I), examples of -V-X include the OMs group, the OTs group, the OTf group, Br, I, -C₂H₂-OMs, -C₂H₂-OTs, -C₂H₂-OTf, -O-C₂H₂-OMs, -O-C₂H₂-OTs, and -O-C₂H₂-OTf.

In the formula (I), carbon marked with * is an asymmetric carbon atom. In the molecular probe precursor of the present invention, the carbon marked with ^{*} may be either a carbon atom in R configuration or a carbon atom in S configuration. Therefore, the molecular probe precursor expressed as the formula (I) may be present as an R body, an S body, a racemic body, or a mixture of the R body and the S body.

As an example of a preferred embodiment of the molecular probe precursor of the present invention, the following compounds may be used. However, the present invention is not limited to these compounds.

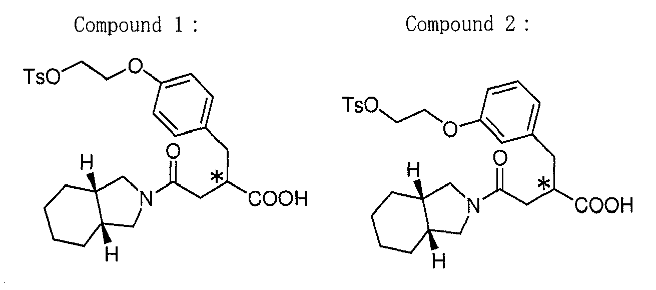

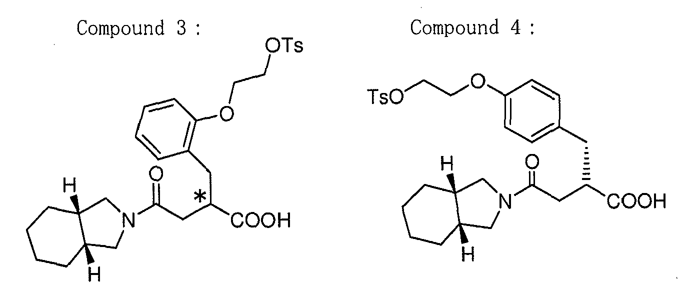

Comparing the compound 1 with the formula (I), V is -O-C₂H₂-, R² is H, and the substituent is in the para position of the benzene ring. Comparing the compound 2 with the formula (I), V is -O-C₂H₂-, R² is H, and the substituent is in the meta position of the benzene ring. Comparing the compound 3 with the formula (I), V is -O-C₂H₂-, R² is H, and the substituent is in the ortho position of the benzene ring. Comparing the compound 4 with the formula (I), V is -O-C₂H₂-, R² is H, the substituent is in the para position of the benzene ring, and the carbon marked with ^{*} is a carbon atom in S configuration. The carboxyl group in each of the compounds 1 to 4 may be protected by a protecting group. That is, R² may be a protecting group, and preferably is a benzyl group. The above example shows the compounds in which R² is H and X is the OTs group. Needless to say, in the present invention, R² and X are not limited to H and the OTs group, respectively.

As described above, the molecular probe precursor of the present invention is used for imaging of pancreatic islets, preferably for noninvasive imaging of pancreatic islets in view of the test and diagnostic applications for humans, and more preferably for imaging of pancreatic islets to quantify the amount of pancreatic islets in view of the same. Moreover, the molecular probe precursor of the present invention is used preferably for imaging of pancreatic islets to prevent, treat, or diagnose diabetes. Such imaging of pancreatic islets may be performed by positron emission tomography (PET).

### [Method for producing molecular probe precursor of the present invention]

A person skilled in the art could synthesize the molecular probe precursor of the present invention expressed as the formula (I), e.g., based on the schemes in Example, which will be described later, and the descriptions of Japanese Patent 2686863 B, Japanese Patent 2686879 B, Domestic re-publication of PCT international application 98-32736 A1, JP 2001-261644 A, etc. Referring to the schemes in Example, other compounds also can be produced.

### [Molecular probe of the present invention]

The molecular probe of the present invention is a compound used for imaging of pancreatic islets and includes a compound expressed as the following formula (II). The molecular probe of the present invention is a compound that can be bound to the pancreatic islets, preferably to the pancreatic β cells, and more preferably to SUR1 on the pancreatic β cells.

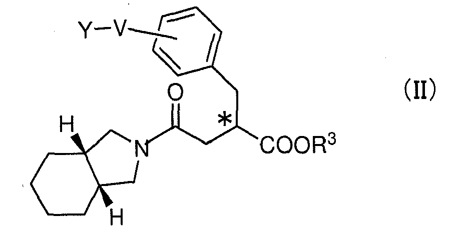

In the formula (II), V and R¹ are the same as those in the formula (I). In the formula (II), R³ represents H, a C₁-C₆ alkyl group, or a C₇-C₁₀ aralkyl group. The C₁-C₆ alkyl group and the C₇-C₁₀ aralkyl group are as described above. R³ is preferably H, a methyl group, or a benzyl group, and more preferably H or the methyl group.

In the formula (II), -V-Y represents a substituent on the benzene ring, and Y represents ¹¹C, ¹⁵O, ¹⁸F, ^{99m}Tc, ¹¹¹In, or ¹²³I, any of which can be selected appropriately in accordance with the intended use of the molecular probe.

### [Method for preparing molecular probe of the present invention]

The molecular probe of the present invention can be prepared by labeling the molecular probe precursor of the present invention in accordance with the imaging method. For example, the molecular probe precursor may be labeled with positron-emitting nuclides such as ¹¹C, ¹⁵O, and ¹⁸F in the case of the PET or with single photon-emitting nuclides such as ^{99m}Tc, ¹¹¹In, and ¹²³I in the case of the SPECT by a known method using, e.g., [¹⁸F] KF, [¹²³I]NH₄I, or the like. If the compound expressed as the formula (I) is labeled by this method, the portion indicated by X is labeled with the above nuclides. However, the labeling method of the present invention is not limited thereto. Thus, another aspect of the present invention relates to a method for producing a molecular probe that includes labeling the molecular probe precursor of the present invention.

In the formula (I), when R² is a protecting group, it is preferable to perform deprotecting after labeling. The deprotecting after labeling can be performed by a known method in accordance with the type of the protecting group. Therefore, the method for producing the molecular probe precursor of the present invention may include deprotecting of the protecting group.

### [Imaging method]

Yet another aspect of the present invention relates to a method for imaging the pancreatic islets. This method includes labeling the molecular probe precursor of the present invention. The imaging method of the present invention also may include imaging the pancreatic islets using the molecular probe of the present invention. The imaging method of the present invention preferably is a method for imaging the pancreatic β cells in view of the test and diagnostic applications. Moreover, the imaging method of the present invention may include deprotecting of the protecting group after labeling. The labeling of the precursor and the deprotecting are as described above. Also, the imaging of pancreatic islets is as described above.

The imaging method of the present invention may include the following: administering the molecular probe of the present invention, which is prepared in the manner as described above, to a subject; and measuring the subject by positron emission tomography (PET) or the like after a predetermined time has passed from the administration of the molecular probe. The PET measurement includes, e.g., capturing an image and measuring the amount of pancreatic islets. The subject may include humans and/or mammals other than humans. The molecular probe may be administered to the subject either locally or systemically. The route of administration can be determined appropriately in accordance with the state of the subject or the like, and may be an intravenous, intraarterial, intradermal, or intraabdominal injection, infusion, or the like. The molecular probe of the present invention preferably is administered with a carrier. Moreover, the molecular probe of the present invention preferably is administered with a pharmaceutical additive such as a carrier. In the present specification, the pharmaceutical additive means any compound that is approved by the Japanese Pharmacopoeia, the United States Pharmacopoeia, the European Pharmacopoeia, or the like. The carrier can be, e.g., an aqueous solvent or a nonaqueous solvent. Examples of the aqueous solvent include a potassium phosphate buffer, physiological saline, a Ringer's solution, and distilled water. Examples of the nonaqueous solvent include polyethylene glycol, vegetable oils, ethanol, glycerol, dimethyl sulfoxide, and propylene glycol. A dose of the molecular probe of the present invention for imaging the pancreatic islets or measuring the amount of pancreatic islets may be, e.g., 1 µg or less. The period of time from the administration to the measurement can be determined appropriately in accordance with a binding time of the molecular probe to the pancreatic islets, the type of the molecular probe, a decomposition time of the molecular probe, or the like.

Yet another aspect of the imaging method of the present invention relates to a method for imaging the pancreatic islets that includes detecting a signal of the molecular probe used for imaging of pancreatic islets of the present invention from a subject to which the molecular probe has been administered. That is, the imaging method of the present invention may include administering the molecular probe of the present invention to a subject and detecting a signal of the molecular probe from the subject after a predetermined time has passed from the administration of the molecular probe. The subject may include humans and/or mammals other than humans. The detection of the signal of the molecular probe includes, e.g., detecting a signal of the radionuclide that is used for labeling of the molecular probe.

### [Method for measuring the amount of pancreatic islets]

Yet another aspect of the present invention relates to a method for measuring the amount of pancreatic islets. This method includes preparing a molecular probe by labeling the molecular probe precursor of the present invention and calculating the amount of pancreatic islets from the result of the imaging of pancreatic islets using the molecular probe. The measuring method of the present invention also may include imaging the pancreatic islets using the molecular probe thus prepared. Moreover, the measuring method of the present invention may include deprotecting after labeling the molecular probe precursor. The labeling and the deprotecting are as described above. Also, the imaging of pancreatic islets is as described above. The calculation of the amount of pancreatic islets from the result of the imaging of pancreatic islets using the molecular probe can be performed, e.g., by analyzing the images obtained from the imaging of pancreatic islets. A person skilled in the art easily can quantify the subject based on the imaging result, e.g., by using a calibration curve or other suitable programs. The measuring method of the present invention preferably is a method for measuring the amount of pancreatic β cells in view of the test and diagnostic applications.

Yet another aspect of the present invention relates to a method for measuring the amount of pancreatic islets that includes the following: detecting a signal of the molecular probe used for imaging of pancreatic islets of the present invention from a subject to which the molecular probe has been administered; and calculating the amount of pancreatic islets from the detected signal of the molecular probe. Moreover, yet another aspect of the present invention relates to a method for measuring the amount of pancreatic islets that includes the following: imaging the pancreatic islets using the molecular probe of the present invention; and calculating the amount of pancreatic islets from the result of the imaging of pancreatic islets.

The measuring method of the present invention further may include presenting the calculated amount of pancreatic islets. The presentation of the calculated amount of pancreatic islets includes, e.g., storing or outputting the calculated amount of pancreatic islets. For outputting, e.g., the calculated amount of pancreatic islets may be displayed on a monitor, printed on paper, or the like.

### [Prevention, treatment, and diagnostic methods of diabetes]

Yet another aspect of the present invention relates to the prevention, treatment, or diagnostic method of diabetes. As described above, the amount of pancreatic islets (particularly the amount of pancreatic β cells) is reduced prior to abnormal glucose tolerance during the development of diabetes, and the treatment of diabetes is already difficult when the dysfunction is detected and the patient is conscious of it. However, with the imaging method and the measuring method of the amount of pancreatic islets using the molecular probe precursor of the present invention, a reduction in the amount of pancreatic islets and/or pancreatic β cells can be found early, so that new prevention, treatment, and diagnostic methods of diabetes can be established. The subject of the prevention, treatment, and diagnosis of diabetes may include humans and/or mammals other than humans. For example, the method for preventing diabetes of the present invention may include checking whether the amount of pancreatic islets tends to decrease by measuring the amount of pancreatic islets at regular intervals. The method for treating diabetes of the present invention may include evaluating the effects of treatment for the subject, including medication and alimentary therapy, while focusing on a change in the amount of pancreatic islets. Moreover, the method for diagnosing diabetes of the present invention may include imaging the pancreatic islets or measuring the amount of pancreatic islets to compare the results with a reference size or amount or to determine the extent of diabetes.

Another preferred aspect of the present invention relates to a method for diagnosing diabetes in the very early stage. This ultra-early diagnosis method for diabetes may include imaging the pancreatic islets and/or measuring the amount of pancreatic islets according to the present invention in a thorough physical examination, a medical examination, etc. and determining the state of the pancreatic islets based on the resultant images of the pancreatic islets and/or the measured amount of pancreatic islets. The method for treating diabetes of the present invention may include imaging the pancreatic islets and/or measuring the amount of pancreatic islets according to the present invention and evaluating the functional recovery of the pancreatic islets based on the resultant images of the pancreatic islets and/or the measured amount of pancreatic islets.

### [Kit]

Yet another aspect of the present invention relates to a kit including the molecular probe precursor of the present invention. Moreover, yet another aspect of the present invention relates to a kit including the molecular probe of the present invention. Embodiments of the kit of the present invention include a kit for preparing the molecular probe of the present invention, a kit for performing the imaging method of the present invention, a kit for performing the measuring method of the amount of pancreatic islets of the present invention, and a kit for preventing, treating, or diagnosing diabetes of the present invention. The kit of the present invention further may include an instruction manual. It is preferable that the instruction manual supports each of the above embodiments.

The kit of the present invention further may include components used to prepare the molecular probe such as a buffer and an osmoregulator and tools used to administer the molecular probe such as a syringe.

Hereinafter, the present invention will be described in more detail by way of examples. However, the present invention is not limited to the following examples.

### [Examples]

### [Preparation of molecular probe precursor and molecular probe]

First, according to the following scheme, a molecular probe precursor (C₃₅H₄₁NO₇S, M. W. 619.77, a pale yellow oily substance) expressed as the following formula (5) was obtained. Comparing the molecular probe precursor, i.e., the compound (5) (S configuration) with the above formula (I), V was -O-C₂H₂-(oxyethylene group), R² was H, X was the OTs group, and -O(CH₂)₂OTs was a substituent in the para position, as shown in the following scheme. In the compound (5), the carboxyl group was protected by a protecting group (Bn).

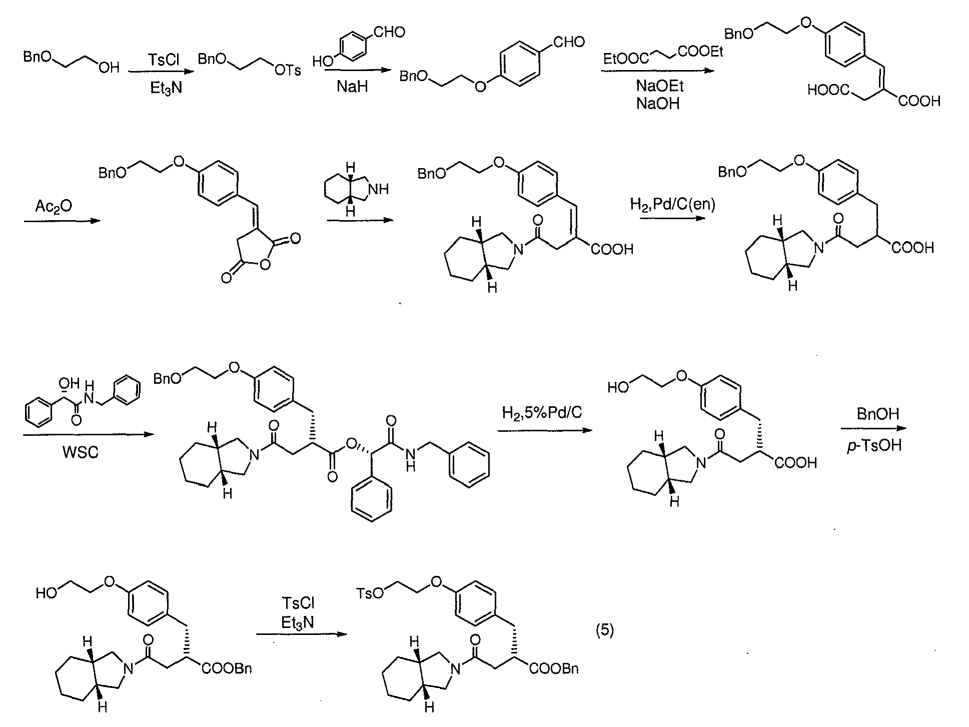

The following is the data of the molecular probe precursor (compound (5)).

IR: 3031, 2927, 2856, 1731, 1639, 1511, 1450, 1359, 1297, 1247, 1176, cm⁻¹
¹H-NMR (400 MHz, CDCl₃): δ7.81 (d, 2H), 7.34 (d, 2H), 7.31-7.22 (m, 5H), 7.00 (d, 2H), 6.65 (d, 2H), 5.16-5.03 (m, 2H), 4.34 (t, 2H), 4.09 (t, 2H), 3.42-3.20 (m, 5H), 2.96-2.91 (m, 1H), 2.80-2.74 (m, 1H), 2.65-2.60 (m, 1H), 2.45 (s, 3H), 2.30-2.20 (m, 2H), 2.19-2.12(m, 1H), 1.55-1.25 (m, 8H)
LC/MS (APCI(+), 40V): 620 (M + H⁺)

Next, the molecular probe precursor (compound (5)) was labeled. Using a labeling reagent including [¹⁸F] KF and Kryptofix (registered trademark) 222 (product name, manufactured by Merck Ltd.,
4,7,13,16,21,24-Hexaoxa-1,10-diazabicyclo[8.8.8]hexacosane), the labeling was performed according to the following scheme. After the labeling, the protecting group (Bn) was removed from the carboxyl group according to the following scheme, thus providing a compound expressed as the following formula (6) that served as a molecular probe. This molecular probe (compound (6)) had generally the same structure as mitiglinide (antidiabetic agent) except that the para position of the benzene ring was substituted with -O(CH₂)₂¹⁸F and the molecular probe (compound (6)) was not a calcium salt.

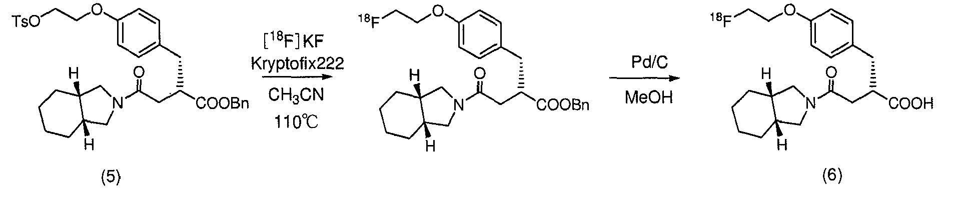

### [Biodistribution]

The biodistribution of mice was measured with the molecular probe (compound (6)) prepared in the manner as described above. First, the molecular probe (102 µCi) was administered intravenously to 6-week-old ddy mice (each of which was male and weighed 25 g) under anesthesia. Then, the organs were extracted 5 minutes, 15 minutes, 35 minutes, 60 minutes, and 120 minutes after the administration, respectively (n = 5). The weight and radioactivity of each of the organs were measured, and the accumulation (% dose/g) of the molecular probe was calculated from the radioactivity per unit weight. FIG. 1 shows an example of the results. FIG. 1A is a graph showing changes in the accumulation of the molecular probe in the individual organs over time. FIG. 1B is an enlarged graph of FIG. 1A.

The accumulation of the molecular probe of this example in the pancreas and the other organs was as shown in FIG. 1.

### [SU receptor binding experiment]

First, according to the following scheme, a compound (7) (C₂₁H₂₈FNO₄, M. W. 377.45, a colorless oily substance) was obtained. The compound (7) had the same structure as the compound (6) except that ¹⁹F was used instead of ¹⁸F.

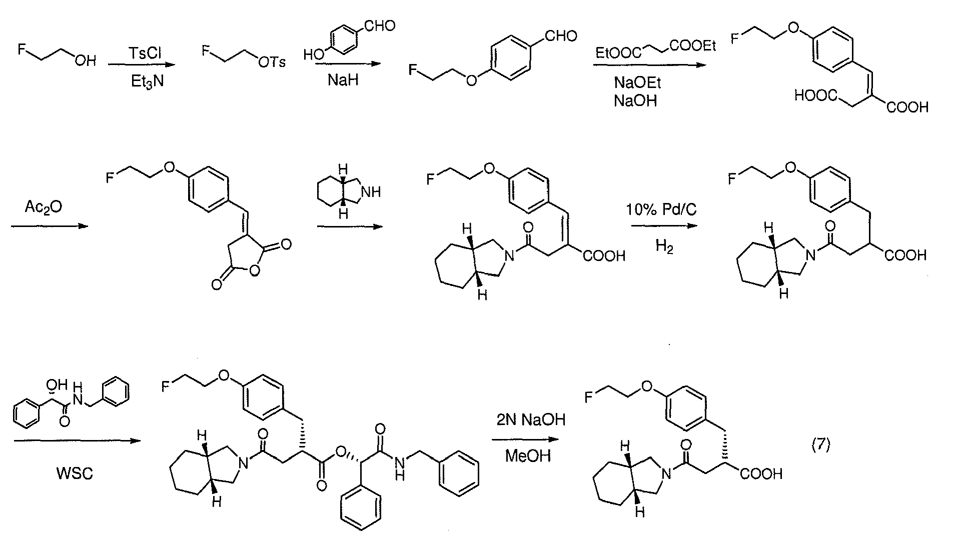

The following is the data of the compound (7).

IR: 2929, 2856, 1727, 1639, 1610, 1511, 1450, 1336, 1299, 1249, 1180, 1112, 1076,1051 cm⁻¹
¹H-NMR (400 MHz, CDCl₃): δ7.11 (d, 2H), 6.87 (d, 2H), 4.75 (dt, 2H), 4.19 (dt, 2H), 3.44-3.36 (m, 2H), 3.35-2.91 (m, 4H), 2.74-2.71 (m, 1H), 2.50-2.43 (m, 2H), 2.22-2.20 (m, 2H), 1.57-1.25 (m, 8H)
LC/MS (APCI(+), 40V): 378 (M + H⁺)

Next, the pancreatic islets isolated from the mice were dispersed into single cells to prepare 1.7 × 10⁵ cells/sample. A solution containing [³H]-glibenclamide, which was obtained by labeling glibenclamide
(4-[2-(5-Chloro-2-methoxybenzoylamino)ethyl]-N-(cyclohexylcarbamoyl)benzenesulfon amide, serving as an SU agent) with ³H, was added to these cells so that the final concentration of the [³H]-glibenclamide was 1.85 pmol/L. Subsequently, a reagent containing the compound (7) was added (final concentration of the compound (7): 1 × 10⁻⁴ to 1 × 10⁻¹² M), and incubation was performed at room temperature for 1 hour. The reaction was stopped by filtration, and the radioactivity was measured with a liquid scintillation analyzer. FIG. 2 shows the result. In FIG. 2, the horizontal axis indicates the concentration of the compound (7) and the vertical axis indicates the intensity of the radioactivity measured.

As shown in FIG. 2, the compound (7) inhibited the bond between the SU receptor and the [³H]-glibenclamide in the concentration-dependent manner. Therefore, the molecular probe expressed as the formula (7) might have a SUR1 binding site. In this case, EC₅₀ was 2.35 × 10⁻⁸ M.

### [Three-dimensional imaging]

The molecular probe (compound (6)) was used to perform three-dimensional imaging of a mouse. The molecular probe (104 µCi) was administered intravenously to a 6-week-old ddy mouse (which was male and weighted 25 g) under anesthesia. Then, the three-dimensional imaging was performed with the following PET apparatus and conditions.
Imaging apparatus: eXplore Vista (product name, manufactured by General Electric Company)
Imaging method: Static Scan
Reconstruction: 2DOSEM (Dynamic OS-EM)

FIG. 3 shows the resultant image of the three-dimensional imaging. The image in FIG. 3 was taken 10 minutes after the administration of the molecular probe (integrating time: 10 minutes). In FIG. 3, the portion indicated by the white circle is near the pancreas.

As shown in FIG. 3, the approximate position of the pancreas could be distinguished noninvasively by using the molecular probe of the present invention expressed as the formula (6). This suggested that the molecular probe precursor and/or the molecular probe of the present invention enabled the three-dimensional noninvasive imaging of pancreatic islets.

### Industrial Applicability

As described above, the present invention is useful, e.g., in the fields of medical care, molecular imaging, and diabetes.

## Claims

1. A precursor of a molecular probe for imaging of pancreatic islets that is expressed as the following formula (I): 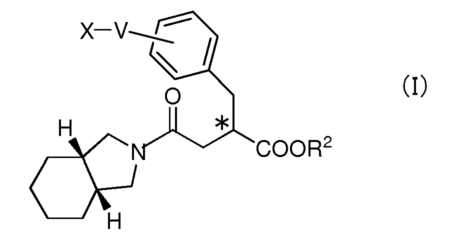 wherein -V-X represents a substituent on a benzene ring, V represents a bond, R¹, or OR¹, R¹ represents a C₁-C₆ alkylene group, R² represents H (hydrogen atom), a C₁-C₆ alkyl group, a C₇-C₁₀ aralkyl group, or a protecting group, X represents a mesylate (OMs) group, a tosylate (OTs) group, a triflate (OTf) group, Br (bromine atom), or I (iodine atom), and carbon marked with ^{*} is an asymmetric carbon atom,
wherein the molecular probe is used for imaging of pancreatic islets.

2. The precursor according to claim 1, wherein in the formula (I), R¹ represents an ethylene group, a propylene group, or a butylene group, R² represents H, a methyl group, or a protecting group, and -V-X is in a meta position or a para position.

3. The precursor according to claim 1 or 2, wherein in the formula (I), the protecting group is a protecting group of a carboxyl group.

4. The precursor according to any one of claims 1 to 3, wherein the precursor is used for noninvasive imaging of pancreatic islets.

5. The precursor according to any one of claims 1 to 4, wherein the precursor is used for imaging of pancreatic islets to quantify an amount of pancreatic islets.

6. The precursor according to any one of claims 1 to 5, wherein the precursor is used for imaging of pancreatic islets to prevent, treat, or diagnose diabetes.

7. The precursor according to any one of claims 1 to 6, wherein the imaging of pancreatic islets is performed by positron emission tomography (PET).

8. A molecular probe for imaging of pancreatic islets that is used for imaging of pancreatic islets and expressed as the following formula (II): 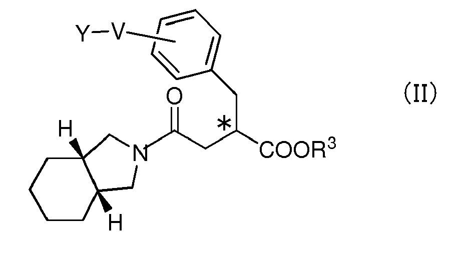 wherein -V-Y represents a substituent on a benzene ring, V represents a bond, R¹, or OR¹, R¹ represents a C₁-C₆ alkylene group, R³ represents H (hydrogen atom), a C₁-C₆ alkyl group, or a C₇-C₁₀ aralkyl group, Y represents ¹¹C, ¹⁵O, ¹⁸F, ^{99m}Tc_{,} ¹¹¹In, or ¹²³I, and carbon marked with ^{*} is an asymmetric carbon atom.

9. The molecular probe according to claim 8, wherein the molecular probe is obtained by labeling the precursor according to any one of claims 1 to 7.

10. A method for imaging pancreatic islets comprising:
labeling the precursor according to any one of claims 1 to 7.

11. A method for measuring an amount of pancreatic islets comprising:
preparing a molecular probe for imaging of pancreatic islets by labeling the precursor according to any one of claims 1 to 7; and
calculating the amount of pancreatic islets from a result of the imaging of pancreatic islets using the molecular probe.

12. A method for producing a molecular probe for imaging of pancreatic islets comprising:
labeling the precursor according to any one of claims 1 to 7.

13. A kit for preparing a molecular probe for imaging of pancreatic islets comprising:
the precursor according to any one of claims 1 to 7.
